# EUROPEAN PATENT APPLICATION

(11) **EP 4 832 378 A1**
(43) Date of publication of application: **28.10.2026**
(21) Application number: 24956091.3
(22) Date of filing: 30.12.2024
(51) Int. Cl.: H04B 10/50

(54) **ULTRA-HIGH-SPEED LASER TRANSMISSION SYSTEM AND CT EXAMINATION DEVICE**

(30) Priority: 29.09.2024 CN 202411369638
(71) Applicant: Beijing Laser Starcom Science & Tecnology Co., Ltd, Beijing 100086 (CN)
(72) Inventor: XUE, Jiang, Beijing 100086 (CN); WU, Shaojun, Beijing 100086 (CN); WANG, Zhe, Beijing 100086 (CN); MIAO, Lianru, Beijing 100086 (CN)
(74) Representative: Kutzenberger Wolff & Partner
(86) International application number: PCT/CN2024/143859
(87) International publication number: WO 2026/065824

(57) **Abstract**

The present application relates to the technical field of medical imaging, and provides an ultra-high-speed laser transmission system and a CT examination device. A photoelectric emission system is suitable for emitting optical signals of at least two different wavelengths. An optical array assembly comprises an even number of emission lenses and a reception lens. The emission lenses are connected to the photoelectric emission system. The emission lenses are arranged on a rotor structure in a circumferential array, and the wavelengths of optical signals passing through two adjacent emission lenses are different. The reception lens is arranged on a stator structure, the field of view of the reception lens covering emergent areas of at least two emission lenses, and the reception lens being used for receiving spatial light beams emitted from two adjacent emission lenses into a single optical fiber for transmission. A photoelectric reception system is connected to the reception lens, and is used for separating signals of different wavelengths so as to perform corresponding signal processing. A signal synchronization processing system is used for performing synchronization processing on received optical signals. The system of the present application has a simple configuration and is convenient to install, which can optimize bandwidth and improve transmission efficiency.

## Description

### CROSS-REFERENCE TO RELATED APPLICATION

The present application claims priority to Chinese patent application No. 2024113696382, filed on September 29, 2024, entitled "Ultra-High-Speed Laser Transmission System and CT Examination Device", which is incorporated herein by reference in its entirety.

### FIELD

The present application relates to the field of medical imaging, and more particularly, to an ultra-high-speed laser transmission system and a computed tomography (CT) examination device.

### BACKGROUND

Computed Tomography (CT) is a medical imaging technology that uses precise X-ray beams and high-sensitivity detector to perform layer-by-layer scanning on a subject. Data obtained by the scanning is processed through a computer to generate high-resolution images of internal cross-sections, coronal planes, or sagittal planes of the subject.

To enhance data transmission rate of a CT system, Chinese patent application No. CN101006925A discloses a CT slip-ring system based on fiber-optic data transmission, and specifically discloses the following contents. When a rotary portion performs rotational scanning, a laser diode and a focusing lens corresponding to the laser diode rotate along with the rotary portion. To receive a beam focused by the focusing lens, a fiber-optic bundle of finite length is provided along a circumference of a stationary portion of the CT system surrounding the rotary portion. The fiber-optic bundle transmits received an optical signal to an optical-to-electrical conversion system, such as a fiber-optic sensor through which the optical signal is converted into an electrical signal and then transmit the electrical signal to an image reconstruction system of the CT system for image reconstruction.

Although the aforementioned solution can improve the data transmission rate of a CT system to a certain extent, since a plurality of focusing lenses are provided at the rotary member, an array of beams is used at a receiving side to receive the optical signal, the light intensity of each individual received beam is relatively weak, and the essentially same optical signal is received by such a great amount of arrays of beams and therefore this solution has the following defects.
(1) A plurality of optical-to-electrical conversion systems are used for reception, two different optical-to-electrical conversion systems may simultaneously receive the same signal, and received same signals need to be processed. Due to optical path differences between the two signals, the two received signals experience varying delays. Whether to select a specific optical signal or attempts to strictly control the optical path difference between the two signals, the signal processing involved is highly complex, which ultimately hinders the enhancement of communication rates.
(2) Power of the received signal is received by a plurality of receivers using a fiber bundle array and energy of any single beam is relatively low to be unable to meet the high-sensitivity requirements necessary for ultra-high-speed operation. Furthermore, a beam array with multiple receivers requires a large number of receiver systems, resulting in a relatively complex system configuration.

### BRIEF SUMMARY

According to the present application, there is provided an ultra-high-speed laser transmission system to solve the aforementioned defects in the related art. The system features a relatively simple configuration and facilitates easy installation. Moreover, its signal processing remains unaffected by increases in transmission rates. The system is capable of optimizing bandwidth utilization to mitigate network congestion and latency, enhancing transmission efficiency.

According to the present application, there is further provided a CT examination device.

The ultra-high-speed laser transmission system of the present application includes an optoelectronic emission system configured to emit at least two optical signals of different wavelengths; an optical array assembly including an even number of emission lenses and a reception lens, where the even number of emission lenses are connected to the optoelectronic emission system and provided at a rotor structure in a circumferential array, wavelengths of optical signals passing through two adjacent emission lenses are different, and the reception lens is provided at a stator structure, a field of view of the reception lens covers emergent areas of at least two emission lenses to receive spatial beams emitted from two adjacent emission lenses into a single optical fiber for transmission; an optoelectronic receiver system connected to the reception lens and configured to demultiplex signals of different wavelengths for performing corresponding signal processing; and a signal synchronization processing system configured to synchronously process received optical signals.

According to the ultra-high-speed laser transmission system of the present application, the optoelectronic emission system includes at least two optoelectronic emission assemblies and a wavelength division multiplexer, where light emitted from each of the at least two optoelectronic emission assemblies is configured to pass through the wavelength division multiplexer and enter the optical array assembly, and the wavelength division multiplexer is configured to multiplex optical signals of different wavelengths onto a single optical fiber for simultaneous transmission to increase an available bandwidth of the optical fiber.

According to the ultra-high-speed laser transmission system of the present application, the wavelength division multiplexer includes a multiplexing unit and a demultiplexing unit, where the multiplexing unit is located at a transmit terminal of the wavelength division multiplexer and configured to combine multiple optical signals of different wavelengths at the transmit terminal and multiplex the multiple optical signals into the same optical fiber for transmission, and the demultiplexing unit is located at a receive terminal of the wavelength division multiplexer and configured to demultiplex optical signals of various wavelengths.

According to the ultra-high-speed laser transmission system of the present application, the ultra-high-speed laser transmission system further includes an optical amplifier located between the multiplexing unit and the demultiplexing unit and configured to amplify energy of the optical signals.

According to the ultra-high-speed laser transmission system of the present application, the optical amplifier includes an optical fiber amplifier.

According to the ultra-high-speed laser transmission system of the present application, the optoelectronic receiver system includes a demultiplexer and at least two optoelectronic receiver assemblies; where the demultiplexer is configured to demultiplex optical signals of different wavelengths, the optoelectronic receiver assemblies are connected to the demultiplexer and configured to perform corresponding processing on demultiplexed optical signals.

According to the ultra-high-speed laser transmission system of the present application, the signal synchronization processing system includes a frame alignment structure configured to perform frame alignment on the signals and a frame counting structure configured to extract a value of a frame count.

According to the present application, there is further provided a CT examination device, including a device body and the ultra-high-speed laser transmission system described above; where the ultra-high-speed laser transmission system is provided at the device body.

In the ultra-high-speed laser transmission system of the present application, two signals emitted from a single optical fiber correspond to two different wavelengths by deploying at least two optical signals of different wavelengths at a transmit terminal. Moreover, these at least two optical signals of different wavelengths are routed respectively into different emission lenses within an optical array assembly, and spatial beams emitted from two adjacent emission lenses are received into a single optical fiber through a reception lens at a receive terminal of the optical array assembly for transmission to effectively reduces both the difficulty of installation and adjustment as well as the overall cost of the ultra-high-speed laser transmission system. Transmitted optical signals are synchronously processed through an optoelectronic receiver system and a signal synchronization processing system, which can not only simplify the overall complexity of the ultra-high-speed laser transmission system but also increase transmission rates from the hundreds of Gbps range to the Tbps range. The ultra-high-speed laser transmission system features a relatively simple configuration and is easy to install. Furthermore, the signal processing remains unaffected by the increased transmission rates, which can optimize bandwidth utilization, mitigate network congestion and latency, and ultimately enhance transmission efficiency.

The CT examination device of the present application shares the same inventive concept as the ultra-high-speed laser transmission system described herein and the CT examination device thus inherently at least possesses all the advantages associated with the aforementioned ultra-high-speed laser transmission system. Moreover, the CT examination device of the present application can be characterized by stable data communication, high transmission efficiency, and a low bit error rate during CT scanning procedures since the CT examination device includes the ultra-high-speed laser transmission system.

### BRIEF DESCRIPTION OF THE DRAWINGS

To illustrate solutions in the present application or the related art more clearly, the drawings to be used in the descriptions of the embodiments or the related art are briefly introduced below. The drawings described below are some embodiments of the present application, and other drawings can be obtained based on o the drawings for those skilled in the art without any creative work.
FIG. 1 is a schematic structural diagram of an ultra-high-speed laser transmission system according to an embodiment of the present application;
FIG. 2 is a schematic structural diagram of an optical array assembly according to an embodiment of the present application;
FIG. 3 is a schematic diagram of an optical path of an optical fiber amplifier according to an embodiment of the present application;
FIG. 4 is a schematic diagram of an optoelectronic emission system and an optoelectronic receiver system according to an embodiment of the present application;
FIG. 5 is a schematic diagram of influence of path delay on a received signal in case that optical signals of same wavelength are used;
FIG. 6 is a schematic diagram of influence of path delay on a received signal in case that optical signals of different wavelengths are used; and
FIG. 7 is a schematic structural diagram of a signal synchronization processing system according to an embodiment of the present application.

### Reference signs:

10, optoelectronic emission system; 20, optical array assembly; 21, stator structure; 211, reception lens; 22, rotor structure; 221, emission lens; 30, optoelectronic receiver system; 40, signal synchronization processing system.

### DETAILED DESCRIPTION

To illustrate objectives, solutions and advantages of the present application, the solutions of the present application are described clearly and completely below in conjunction with the drawings in the application. The described embodiments are part of the embodiments of the application, not all of them. All other embodiments obtained by those skilled in the art based on the embodiments of the present application without any creative work belong to the scope of the present application.

In the description of embodiments of the present application, it should be noted that, unless otherwise explicitly specified and defined, the terms "connected to" and "connected" shall be understood broadly. For example, it may be either fixedly connected or detachably connected, or may be integrally connected; it may be either mechanically connected, or electrically connected; it may be either directly connected, or indirectly connected through an intermediate medium. The specific meanings of the terms above in embodiments of the present application may be understood by those skilled in the art in accordance with specific conditions.

In the embodiments of the present application, unless otherwise clearly stated and defined, the first feature being located "on" or "under" the second feature means that the first feature is in direct contact with the second feature or the first feature is in contact with the second feature by an intermediate medium. In addition, the first feature is "on," "above" and "over" the second feature can refer to that the first feature is directly above or obliquely above the second feature, or simply refer to that the level of the first feature is higher than that of the second feature. The first feature is "under", "below" and "beneath" the second feature can refer to that the first feature is directly below or obliquely below the second feature, or simply refer to that the level of the first feature is lower than that of the second feature.

In the description of the present specification, description with reference to the terms "one embodiment," "some embodiments," "an example," "specific example," "some examples" and the like, refers to that specific features, structures, materials or characteristics described in combination with an embodiment or an example are included in at least one embodiment or example of the embodiments of the present application. In the present specification, the schematic expressions of the above terms are not necessarily directed to the same embodiment or example. Also, the described specific features, structures, materials or characteristics can be combined in any one or more embodiments or examples in a suitable manner. In addition, those skilled in the art may combine the different embodiments or examples described in this specification, as well as the features of the different embodiments or examples, without conflicting each other.

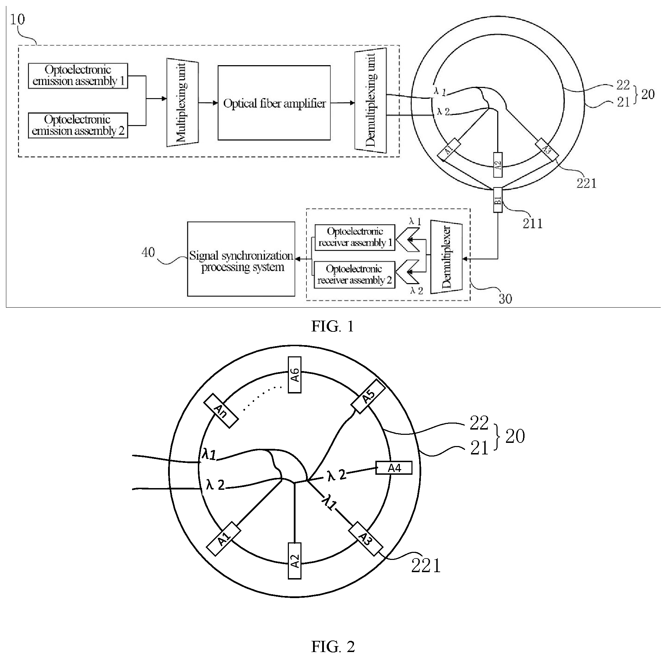

FIG. 1 is a schematic structural diagram of an ultra-high-speed laser transmission system according to an embodiment of the present application; and FIG. 2 is a schematic structural diagram of an optical array assembly according to an embodiment of the present application.

Referring to FIG. 1 and FIG. 2, according to an embodiment of the present application, there is provided an ultra-high-speed laser transmission system. The ultra-high-speed laser transmission system is primarily intended for use in CT monitoring device and may also be applied in other fields, such as video streaming, big data cloud computing, and etc.

The ultra-high-speed laser transmission system includes an optoelectronic emission system 10, an optical array assembly 20, an optoelectronic receiver system 30, and a signal synchronization processing system 40.

The optoelectronic emission system 10 may emit at least two optical signals of different wavelengths. That is, the optoelectronic emission system 10 at least may emit two optical signals of different wavelengths and may also emit three optical signals of different wavelengths as required. Transmission signals carried by at least two optical signals of different wavelengths are identical. In the embodiment of the present application, the description is made by taking the emission of two optical signals of different wavelengths by the optoelectronic emission system 10 as an illustrative example. Additionally, the optoelectronic emission system 10 is further configured to demultiplex amplified optical signals and route the demultiplexed optical signals into the optical array assembly 20.

The optical array assembly 20 is connected to the optoelectronic emission system 10. The optical array assembly 20 includes an even number of emission lenses 221 and a reception lenses 211. The even number of emission lenses 221 are provided at a rotor structure 22 in a circumferential array. Each emission lens 221 serves as a collimating lens configured to collimate light entering from a spectrometer and optical signals passing through two adjacent emission lenses 221 have different wavelengths. The reception lens 211 is provided at a stator structure 21, a field of view of the reception lens 211 covers emergent areas of at least two emission lenses 221 to receive spatial beams emitted from two adjacent emission lenses 221 into a single optical fiber for transmission. The number of the reception lens 211 may be one or may also be multiple. The rotor structure 22 rotates relative to the stator structure 21 and the rotor structure 22 is provided at a slip ring through which power is supplied to the rotor structure 22.

The optoelectronic receiver system 30 is connected to the reception lens 211 and is configured to demultiplex signals of different wavelengths for performing corresponding signal processing. The signal synchronization processing system 40 is configured to synchronously process received optical signals.

It is to be understood that in the ultra-high-speed laser transmission system of the present application, two signals emitted from a single optical fiber correspond to two different wavelengths by deploying at least two optical signals of different wavelengths at a transmit terminal. Moreover, these at least two optical signals of different wavelengths are routed respectively into different emission lenses 221 within an optical array assembly 20, and spatial beams emitted from two adjacent emission lenses are received into a single optical fiber through a reception lens 211 at a receive terminal of the optical array assembly 20 for transmission to effectively reduces both the difficulty of installation and adjustment as well as the overall cost of the ultra-high-speed laser transmission system. Transmitted optical signals are synchronously processed through an optoelectronic receiver system 30 and a signal synchronization processing system 40, which can not only simplify the overall complexity of the ultra-high-speed laser transmission system but also increase transmission rates from the hundreds of Gbps range to the Tbps range. The ultra-high-speed laser transmission system features a relatively simple configuration and is easy to install. Furthermore, the signal processing remains unaffected by the increased transmission rates, which can optimize bandwidth utilization, mitigate network congestion and latency, and ultimately enhance transmission efficiency.

Referring further to FIG. 1, in some embodiments of the present application, the optoelectronic emission system 10 includes at least two optoelectronic emission assemblies and a wavelength division multiplexer, where each optoelectronic emission assembly is a transmitter. Light emitted from each of the optoelectronic emission assemblies passes through the wavelength division multiplexer and enters the optical array assembly 20; and the wavelength division multiplexer is configured to multiplex optical signals of different wavelengths onto a single optical fiber for simultaneous transmission to increase an available bandwidth of the optical fiber.

In the ultra-high-speed laser transmission system, the wavelength division multiplexer is installed in an optical fiber between the transmit terminal and the receive terminal and configured to combine optical signals of different wavelengths transmitted from at least two distinct optoelectronic emission assemblies into a single wavelength-multiplexed optical signal for transmission over a single optical fiber. A wavelength division demultiplexer is used at the receive terminal to demultiplex the optical signals and then individually transmit the demultiplexed optical signals to the optical array assembly 20.

For instance, two optoelectronic emission assemblies are designated as an optoelectronic emission assembly 1 and an optoelectronic emission assembly 2. The optoelectronic emission assembly 1 emits an optical signal of a wavelength λ1, while the optoelectronic emission assembly 2 emits an optical signal of a wavelength λ2. These optical signals λ1 and λ2 pass through the wavelength division multiplexer and are transmitted to the optical array assembly 20. Here, a single reception lens 211 receives the spatial optical beams emitted from two adjacent emission lenses 221 into a single optical fiber. By deploying the system with two wavelength intervals at the transmit terminal, the two signals received within this single optical fiber correspond to two signals of different wavelengths. These two optical signals of different wavelengths are then routed respectively into different optoelectronic receiver systems 30 and processed synchronously through the signal synchronization processing system 40. The processing process is unaffected by increases in data rate, which can optimize bandwidth, reduce network congestion and latency while enhancing the transmission efficiency of outgoing signals.

Referring further to FIG. 1, in some embodiments of the present application, the wavelength division multiplexer includes a multiplexing unit and a demultiplexing unit. The multiplexing unit is located at a transmit terminal of the wavelength division multiplexer and configured to combine multiple optical signals of different wavelengths carrying various forms of information) through a multiplexer at the transmit terminal and multiplex the multiple optical signals into the same optical fiber for transmission. The demultiplexing unit is located at a receive terminal of the wavelength division multiplexer and configured to demultiplex optical signals of various wavelengths through a demultiplexer.

Demultiplexed optical signals λ1 and λ2 are routed to the optical array assembly 20. The spatial optical beams emitted from two adjacent emission lenses 221 are received into a single optical fiber through a single reception lens 211 provided at the stator structure 21 of the optical array assembly 20. The two signals received within this single optical fiber correspond to two signals of different wavelengths. These two optical signals of different wavelengths are demultiplexed by a demultiplexer and then routed respectively into different optoelectronic receiver systems 30 to recover original signals. The original signals are processed synchronously through the signal synchronization processing system 40. The processing process is unaffected by increases in data rate, which can optimize bandwidth, reduce network congestion and latency while enhancing the processing efficiency of signals.

Referring further to FIG. 1, in some embodiments of the present application, the ultra-high-speed laser transmission system further includes an optical amplifier located between the multiplexing unit and the demultiplexing unit. Based on the principle of stimulated emission of light, the optical amplifier achieves signal amplification by converting the energy of pump light into the energy of the optical signal. The intensity of the optical signal is enhanced to improve transmission distance and quality of the signals.

The optical amplifier includes a semiconductor amplifier and an optical fiber amplifier. The semiconductor amplifier is further classified into a resonant type and a traveling-wave type. The optical fiber amplifier is classified into a rare-earth-doped optical fiber amplifier and a nonlinear optical fiber amplifier.

The embodiments of the present application are illustrated using a rare-earth-doped optical fiber amplifier as an example, specifically, an optical fiber is doped with rare-earth ions (such as erbium, praseodymium, thulium, etc.) acting as the laser-active medium. The gain bandwidth varies for each specific dopant. An erbium-doped optical fiber amplifier possesses a broad gain band, covering the S, C, and L bands; a thulium-doped optical fiber amplifier operate within the S-band; and a praseodymium-doped optical fiber amplifier has a gain band around 1310 nm.

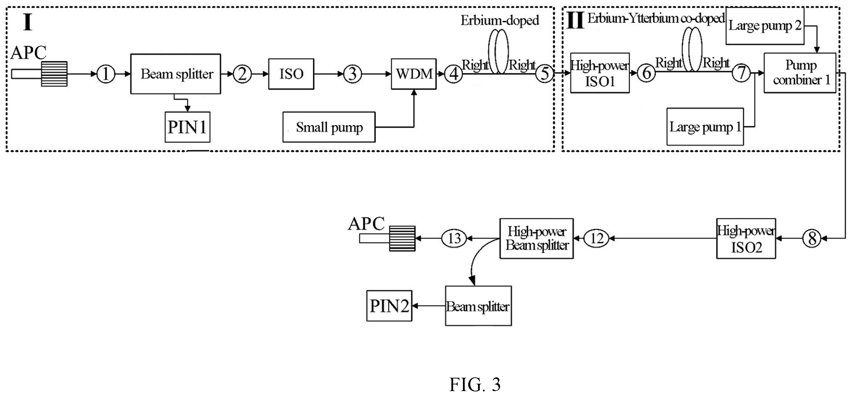

FIG. 3 is a schematic diagram of an optical path of an optical fiber amplifier according to an embodiment of the present application.

Referring to FIG. 3, the optical fiber amplifier is configured to amplify a low-power output optical signal into a high-power output optical signal to support attenuation over longer distances and ensure that the signal received at the receive terminal falls within an acceptable power range. The optical fiber amplifier may have an output power of up to x Watts (xW) while maintaining a noise figure of less than 5 dB.

A single-stage erbium-doped fiber amplifier has an output power of 22 dBm approaching its practical limit and can maintain a low noise figure. Consequently, the optical path design for a high-power optical amplifier typically employs a cascaded, two-stage amplification structure, as illustrated in FIG. 3. A first amplification stage is referred to as the pre-amplifier, and an erbium-doped fiber pumped by a 976 nm single-mode source is used to provide an initial power boost to the input signal while keeping the noise figure controlled below 4.5 dB. In a second amplification stage, double-clad amplification technology with a 940 nm multi-mode pump as the excitation source and a double-clad erbium-ytterbium co-doped fiber as the gain medium is used. Structurally, this stage adopts a backward-pumping configuration to enhance pump conversion efficiency.

It should be noted that, in the design of this two-stage optical amplification path, power consumption and overall system reliability need to be considered besides considering the conversion efficiency of the amplification path.

Referring further to FIG. 1, the photoelectric receiver system 30 includes a demultiplexer and at least two photoelectric receiver assemblies, where each of the photoelectric receiver assemblies is a receiver. The demultiplexer is configured to demultiplex the optical signals received from the single reception lens 211 to recover optical signals λ1 and λ2. The optical signal λ1 is routed to its corresponding optoelectronic reception assembly 1 for being processed while optical signal λ2 is routed to its corresponding optoelectronic reception assembly 2 for being processed.

The reception lens 211 of the optical array assembly 20 functions as a multiplexer; together with the demultiplexer of the photoelectric receiver system 30 forms a wavelength division multiplexing system. That is, the ultra-high-speed laser transmission system of the embodiments of the present application employs a multi-stage wavelength division multiplexing system to increase the available bandwidth of the optical fiber and enhance the transmission rate of signals.

Referring further to FIG. 1 and FIG. 2, an even number of emission lenses 221 are provided at the rotor structure 22 in a circumferential array, that is, these even number of emission lenses 221 are distributed uniformly at the rotor structure 22. The optical signals transmitted from any two adjacent emission lenses 221 have different wavelengths and the transmitted optical signals are identical.

For instance, emission lenses 221, designated as A1, A2, A3, A4, A5, An, etc. are fixed at regular intervals at the rotor structure 22. Lens B1 is a reception lens 211 fixedly provided at the stator structure 21. The field of view of the reception lens 211 is determined by its positional relationship with the two adjacent emission lenses 221. Specifically, structural parameters of the reception lens 211 can be configured based on the positions of these adjacent emission lenses 221 to ensure that the field of view of the reception lens covers the emergent areas of both adjacent emission lenses 221 and guarantee that the optical signals emitted from these two adjacent lenses are simultaneously received under all operating conditions.

Optical signal amplified by an optical fiber amplifier within the wavelength division multiplexer is demultiplexed through a demultiplexer into two optical signals λ1 and λ2. An optical signal of a wavelength λ1 is split through a 2-to-1 optical fiber and then enters emission lenses A1 and A3, respectively while an optical signal of a wavelength λ2 enters emission lens A2. Subsequently, these three optical signals are collimated by emission lenses A1, A2, and A3, respectively, and are then emitted radially outward from the rotor structure 22. The field of view of reception lens B1 expands radially outward from the rotor to completely cover the entire emission range spanning from emission lens A1 to emission lens A3. As the rotor structure 22 rotates, the reception lens B1 receives a combined optical signal of optical signals λ1 and λ2 collimated and emitted from the emission lenses A1 through A3. These signals are multiplexed by the reception lens B1 into a single optical fiber and is then input to a wavelength division demultiplexter (i.e., a demultiplexer) for being demultiplexed. The demultiplexd optical signal λ1 is subsequently routed to the photoelectric reception assembly 1 for being processed, while the demultiplexd optical signal λ2 is routed to the photoelectric reception assembly 2 for being processed. These optical signals λ1 and λ2 are synchronously processed through the signal synchronization processing system 40.

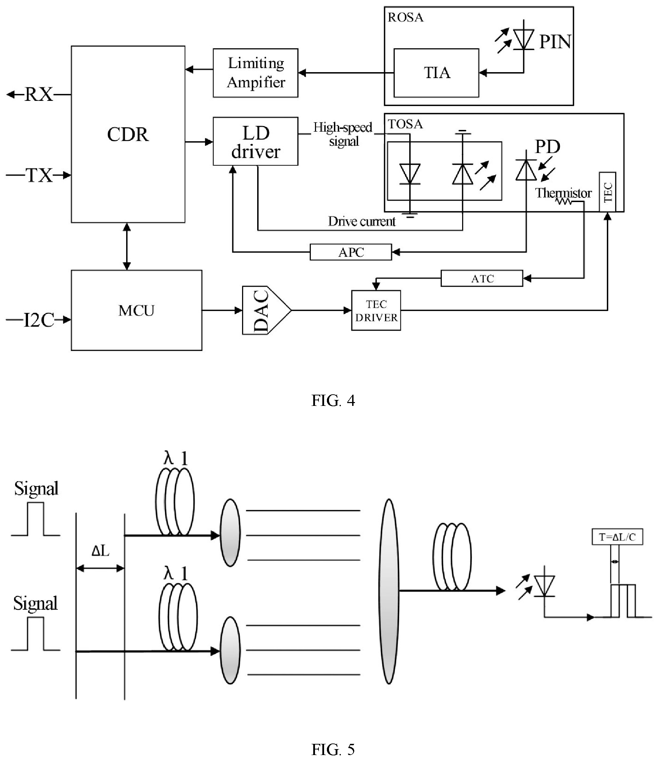

FIG. 4 is a schematic diagram of an optoelectronic emission system and an optoelectronic receiver system according to an embodiment of the present application.

Referring to FIG. 4, TX represents an electrical signal input to the transmit terminal of the ultra-high-speed laser transmission system. Clock recovery and regeneration is performed on the electrical signal through a clock and data recovery (CDR) circuit and output to a laser driver (LDDRIVER) to amplify the low-amplitude signal into a high-amplitude signal. The laser driver then modulates this electrical signal onto an optical signal. A transmitter optical subassembly (TOSA) subsequently emits the optical signal.

A receiver optical sub-assembly (ROSA) is configured to convert the received optical signal back into an electrical signal using a PIN. The electrical signal is amplified through a transimpedance amplifier (TIA) and is subjected to clock and data recovery via a CDR circuit; once being regenerated and recovered, the data is transmitted to the RX.

TEC is a semiconductor refrigerator acting as a controller to regulate the temperature of a laser die, while the APC is the automatic power control circuit for the laser. MCU is a microcontroller unit, also known as a single-chip microcomputer or microcontroller is primarily used to connect with interfaces and execute corresponding control processing tasks.

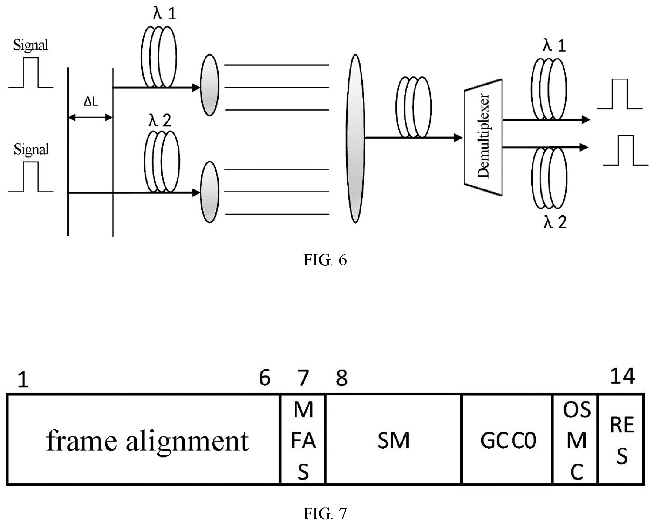

FIG. 5 is a schematic diagram of influence of path delay on a received signal in case that optical signals of same wavelength are used. FIG. 6 is a schematic diagram of influence of path delay on a received signal in case that optical signals of different wavelengths are used.

Referring to FIG. 5 and FIG. 6, for the ultra-high-speed laser transmission system of the embodiment of the present application, the issue of signal interference is illustrated by comparing influence of path delay on a received signal in case that optical signals of same wavelength are used with influence of path delay on a received signal in case that optical signals of different wavelengths are used.

Referring specifically to FIG. 5: since the even number of emission lenses 221 must be provided at the rotor structure 22, and the signal from each emission lens 221 is transmitted via an optical fiber, each emission lens 221 corresponds to a specific optical fiber. Consequently, the lengths of these optical fibers may vary. During the transmission process of the ultra-high-speed laser transmission system, these optical fiber of unequal lengths can lead to signal transmission delays and result in mutual signal interference when the signals from different channels arrive at the receive terminal end simultaneously, which easily results in signal reception failure.

As shown in FIG. 6, the wavelength division multiplexing is used in the ultra-high-speed laser transmission system of the embodiment of the present application. When optical signals of two different wavelengths, that is, an optical signal λ1 and an optical signal λ2, enter the receiver simultaneously via different paths, a wavelength division demultiplexer is used to demultiplex these two signals with different delays, and the issue of mutual interference between the two signals does not occur.

FIG. 7 is a schematic structural diagram of a signal synchronization processing system 40 according to an embodiment of the present application.

Referring to FIG. 7, signal synchronization processing system 40 includes a frame alignment structure configured to perform frame alignment on signals and a frame counting structure configured to extract a value of a frame count.

In the ultra-high-speed laser transmission system of the embodiment of the present application, baseband signals transmitted via the two optical signals of different wavelengths are identical. During optical reception, a scenario where single optical signal is received or two optical signals are received simultaneously occurs as the rotor structure 22 rotates. As the rotor structure 22 continues to rotate, a path in which the optical signal is firstly received rotates gradually out of the field of view of the reception lens 211, while a newly routed optical signal is transitioned from a weak signal to a strong signal. Consequently, synchronization and determination need to be performed on the received signals to ensure that a single complete signal can be synthesized from the two received signals without interrupting the overall signal transmission and subsequently forwarded to the subsequent processing circuit.

As shown in FIG. 7, since the two optical signals share an identical frame header signal, parallel synchronization is performed on their respective frame headers. Once the signals are synchronized, a relative arrival order of the two optical signals is determined. Subsequently, determination is performed based on channel quality metrics, such as the signal-to-noise ratio to determine whether the quality of the earlier-arriving signal is sufficient to support reception of output. If it is judged that the signal quality of the earlier-arriving signal falls below a predetermined threshold, i.e., the signal quality of the earlier arriving signal is less than a signal threshold, then it is necessary for the system to switch to the later-arriving optical signal path for reception. To prevent loss or duplicated reception of data, the received data signals are synchronized and whether received frames are duplicated or lost or not is then determined based on a count within data frames.

Frame alignment is a fixed numerical sequence characterized by strong autocorrelation properties. Frame alignment may be performed while the multi-frame alignment signal (MFAS) serves as a frame counter. Once frame alignment is completed, the value of the frame counter may be extracted to determine a specific sequence number of the currently received frame. This facilitates determining whether received frames are duplicated or lost or not for two signals received in parallel ensuring the reconstruction of a complete transmission sequence and enabling signal synchronization processing.

In the ultra-high-speed laser transmission system of the embodiment of the present application, the optoelectronic emission assembly emits signals of various wavelengths through a plurality of transmitters, converts acquired signals into high-speed optical signals and forwards the high-speed optical signals to an optical amplifier. The amplified optical signals are then demultiplexed through a wavelength division multiplexing system into optical signals of different wavelengths. These optical signals of different wavelengths are routed to an optical array assembly, respectively. Upon being received through the reception lens (211), spatial beams enter a demultiplexter to be demultiplexed into two optical signals of different wavelengths and are routed into different optoelectronic receiver assemblies for being processed. Finally, two received signals are synchronously processed through the signal synchronization processing system (40) and the final data are transmitted to a subsequent processing stage.

The ultra-high-speed laser transmission system of the embodiment of the present application amplifies the optical signals by providing the optical fiber amplifiers, enabling the optical signals to support significantly longer transmission distances. Furthermore, the simultaneous transmission of identical information across multiple wavelengths is enabled using the wavelength division multiplexing system to ensure that the signals are reliably received at the receive terminal to solve the issue of optical path differences in multi-path transmission without optical path calibration and the ultra-high-speed laser transmission system is easy to install. Additionally, the reception lens 211 is provided at the receive side of the optical array assembly 20 to reduce both the complexity of alignment and adjustment, as well as the overall cost of the ultra-high-speed laser transmission system. Finally, the signal synchronization processing technology is employed to simplify the complexity of the entire ultra-high-speed laser transmission system as well as to increase the transmission rate from the hundreds of Gbps range to the Tbps range without requiring significant modifications to an original architecture of the system.

According to an embodiment of the present application, there is further provided a CT examination device, including a device body and the ultra-high-speed laser transmission system described; where the ultra-high-speed laser transmission system is provided at device body.

The CT examination device of the present application shares the same inventive concept as the ultra-high-speed laser transmission system described herein and the CT examination device thus inherently at least possesses all the advantages associated with the aforementioned ultra-high-speed laser transmission system. Moreover, the CT examination device of the present application can be characterized by stable data communication, high transmission efficiency, and a low bit error rate during CT scanning procedures since the CT examination device includes the ultra-high-speed laser transmission system.

Finally, it should be noted that the above embodiments are only used to explain the solutions of the present application, and are not limited thereto; although the present application has been described in detail in conjunction with the foregoing embodiments, it should be understood by those skilled in the art that they can still modify the solutions documented in the foregoing embodiments and make equivalent substitutions to a part of the features; and these modifications and substitutions do not make the essence of the corresponding solutions depart from the scope of the solutions of various embodiments of the present application.

## Claims

1. An ultra-high-speed laser transmission system, comprising:
an optoelectronic emission system configured to emit at least two optical signals of different wavelengths;
an optical array assembly comprising an even number of emission lenses and a reception lens, the even number of emission lenses being connected to the optoelectronic emission system and provided at a rotor structure in a circumferential array, wavelengths of optical signals passing through two adjacent emission lenses being different, the reception lens being provided at a stator structure, a field of view of the reception lens covering emergent areas of at least two emission lenses to receive spatial beams emitted from two adjacent emission lenses into a single optical fiber for transmission;
an optoelectronic receiver system connected to the reception lens and configured to demultiplex signals of different wavelengths for performing corresponding signal processing; and
a signal synchronization processing system configured to synchronously process received optical signals.

2. The ultra-high-speed laser transmission system of claim 1, wherein the optoelectronic emission system comprises at least two optoelectronic emission assemblies and a wavelength division multiplexer, light emitted from each of the at least two optoelectronic emission assemblies being configured to pass through the wavelength division multiplexer and enter the optical array assembly, and the wavelength division multiplexer being configured to multiplex optical signals of different wavelengths onto a single optical fiber for simultaneous transmission to increase an available bandwidth of the optical fiber.

3. The ultra-high-speed laser transmission system of claim 2, wherein the wavelength division multiplexer comprises a multiplexing unit and a demultiplexing unit, the multiplexing unit being located at a transmit terminal of the wavelength division multiplexer and configured to combine multiple optical signals of different wavelengths at the transmit terminal and multiplex the multiple optical signals into the same optical fiber for transmission; the demultiplexing unit being located at a receive terminal of the wavelength division multiplexer and configured to demultiplex optical signals of various wavelengths.

4. The ultra-high-speed laser transmission system of claim 3, further comprising an optical amplifier located between the multiplexing unit and the demultiplexing unit and configured to amplify energy of the optical signals.

5. The ultra-high-speed laser transmission system of claim 4, wherein the optical amplifier comprises an optical fiber amplifier.

6. The ultra-high-speed laser transmission system of any of claims 1 to 5, wherein the optoelectronic receiver system comprises a demultiplexer and at least two optoelectronic receiver assemblies; and
wherein the demultiplexer is configured to demultiplex optical signals of different wavelengths, and the optoelectronic receiver assemblies are connected to the demultiplexer and configured to perform corresponding processing on demultiplexed optical signals.

7. The ultra-high-speed laser transmission system of any of claims 1 to 5, wherein the signal synchronization processing system comprises a frame alignment structure configured to perform frame alignment on signals and a frame counting structure configured to extract a value of a frame count.

8. A computed tomography (CT) examination device, comprising a device body and the ultra-high-speed laser transmission system of any of claims 1 to 7, wherein the ultra-high-speed laser transmission system is provided at the device body.
